(19) Europäisches Patentamt
European Patent Office
Office européen des brevets

(11) Veröffentlichungsnummer: **0 110 243**
**A1**

(12) # EUROPÄISCHE PATENTANMELDUNG

(21) Anmeldenummer: **83111477.2**

(22) Anmeldetag: **17.11.83**

(51) Int. Cl.³: **C 07 D 491/153**, C 07 D 495/14, A 01 N 43/90 // (C07D491/153, 311/00, 311/00, 231/00), (C07D495/14, 335/00, 311/00, 231/00)

(30) Priorität: **26.11.82 DE 3243714**

(43) Veröffentlichungstag der Anmeldung: **13.06.84 Patentblatt 84/24**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Stegelmeier, Hartmut, Dr., Meide 1d, D-4010 Hilden (DE)**
Erfinder: **Brandes, Wilhelm, Dr., Eichendorffstrasse 3, D-5653 Leichlingen (DE)**

(54) **Pyrano-pyrazol-Derivate.**

(57) Die Erfindung betrifft neue Pyrano-pyrazol-Derivate, ein Verfahren zu ihrer Herstellung und ihre Verwendung als Schädlingsbekämpfungsmittel.

Die neuen Verbindungen der Formel (I)

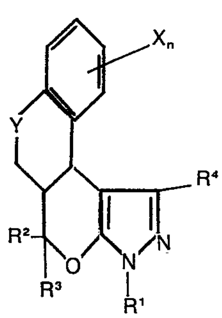

(I)

werden erhalten, wenn man substituierte aromatische Aldehyde der Formel (II)

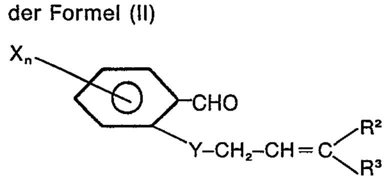

(II)

mit Pyrazolinonen der Formel (III)

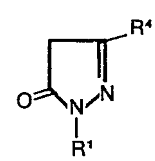

(III)

in welchen Formeln $R^1$ bis $R^4$, Y, X und n die in der Beschreibung gegebene Bedeutung haben, in Gegenwart eines Verdünnungsmittels und gegebenenfalls in Gegenwart eines Katalysators umsetzt.

Die Verbindungen sind für den Gebrauch als Pflanzenschutzmittel geeignet und zeichnen sich insbesondere durch eine hohe fungizide Wirksamkeit aus.

EP 0 110 243 A1

BAYER AKTIENGESELLSCHAFT
Zentralbereich
Patente, Marken und Lizenzen

5090 Leverkusen, Bayerwerk
24.11.82
Bas/Kü-c
Ia

Pyrano-pyrazol-Derivate

Die Erfindung betrifft neue Pyrano-pyrazol-Derivate, ein Verfahren zu ihrer Herstellung sowie ihre Verwendung als Schädlingsbekämpfungsmittel.

Es ist bereits bekannt, daß neben einer Reihe weiterer Substanzklassen auch bestimmte Heterocyclen, wie z.B. N-Trichlormethylthio-tetrahydrophthalimid, fungizide Eigenschaften besitzen (vgl. R. Wegler, "Chemie der Pflanzenschutz- und Schädlingsbekämpfungsmittel" Band 2, S. 108, Springer Verlag Berlin/Heidelberg/New York 1970).

Die Wirksamkeit dieser Verbindungen ist jedoch, insbesondere bei niedrigen Aufwandmengen und -konzentrationen, nicht immer völlig zufriedenstellend.

Es wurden neue Pyrano-pyrazol-Derivate der allgemeinen Formel (I),

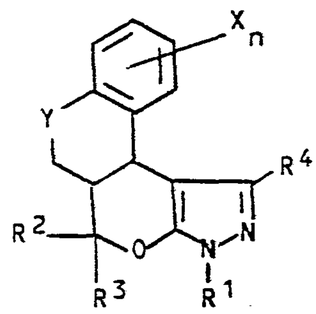

(I)

in welcher

$R^1$ für Wasserstoff, für Alkyl, für Alkanoyl, für Alkoxycarbonyl, für gegebenenfalls substituiertes Phenyl oder für gegebenenfalls substituiertes Phenylalkyl steht,

$R^2$ und $R^3$ gleich oder verschieden sein können und für Wasserstoff,für Alkyl oder für gegebenenfalls substituiertes Phenyl stehen,

$R^4$ für Cyano, für Alkyl, für Alkoxycarbonyl oder für gegebenenfalls sustituiertes Phenyl steht,

Y für Sauerstoff, Schwefel oder die Methylengruppe steht,

X für Halogen, Alkyl, Alkoxy, Alkylthio, Halogenalkyl, Dialkylamino, Nitro oder Cyano steht und

n für eine Zahl von 0 bis 3 steht,

gefunden.

Weiterhin wurde gefunden, daß man die neuen Pyrano-pyrazol-Derivate der allgemeinen Formel (I),

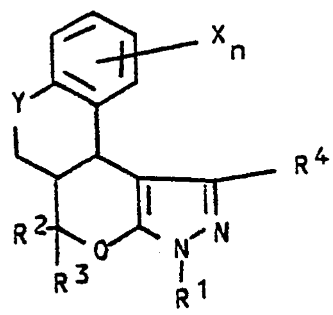

(I)

in welcher

$R^1$ für Wasserstoff, für Alkyl, für Alkanoyl, für Alkoxycarbonyl, für gegebenenfalls substituiertes Phenyl oder für gegebenenfalls substituiertes Phenylalkyl steht,

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls subtituiertes Phenyl steht,

Y für Sauerstoff, Schwefel oder die Methylengruppe steht,

X für Halogen, Alkyl, Alkoxy, Alkylthio, Halogenalkyl, Dialkylamino, Nitro oder Cyano steht und

n für eine Zahl von 0 bis 3 steht,

erhält, wenn man substituierte aromatische Aldehyde der allgemeinen Formel (II),

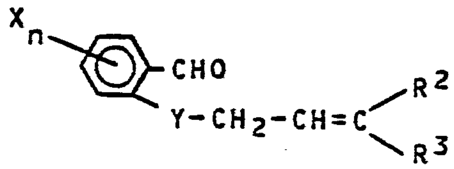

(II)

in welcher

$R^2$, $R^3$, X, Y und n die oben angegebene Bedeutung haben,

mit Pyrazolinonen der allgemeinen Formel (III),

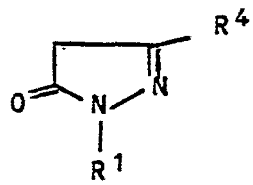

(III)

in welcher

$R^1$ und $R^4$ die oben angegebene Bedeutung haben,

in Gegenwart eines Verdünnungsmittels und gegebenenfalls in Gegenwart eines Katalysators umsetzt.

Die neuen Pyrano-pyrazol-Derivate der allgemeinen Formel (I) weisen starke fungizide Eigenschaften auf. Dabei zeigen die erfindungsgemäßen Verbindungen der Formel (I) überraschenderweise eine bessere fungizide Wirksamkeit als die nach dem Stand der Technik bekannte Verbindung N-Trichlormethylthio-tetrahydrophthalimid, welches eine wirkungsmäßig naheliegende Verbindung ist.

Die erfindungsgemäßen Stoffe stellen somit eine Bereicherung der Technik dar.

Die erfindungsgemäßen Pyrano-pyrazol-Derivate sind durch die Formel (I) allgemein definiert.

Bevorzugt sind diejenigen Verbindungen der Formel (I), bei denen

$R^1$ für Wasserstoff, für geradkettiges oder verzweigtes Alkyl, Alkanoyl oder Alkoxycarbonyl mit jeweils bis zu 4 Kohlenstoffatomen im Alkylteil, für gegebenenfalls einfach oder mehrfach, gleich oder verschieden substituiertes Phenyl oder für gegebenenfalls einfach oder mehrfach, gleich oder verschieden substituiertes Phenylalkyl mit 1 bis 2 Kohlenstoffatomen im Alkylteil steht, wobei als Phenylsubstituenten jeweils in Betracht kommen: Halogen, Cyano, Nitro, Alkyl mit bis zu 4 Kohlenstoffatomen, Alkoxy, Alkylthio oder Dialkylamino mit 1 bis 2 Kohlenstoffatomen in den jeweiligen Alkylteilen, sowie Halogenalkyl mit 1 oder 2 Kohlenstoffatomen und bis zu 5 gleichen oder verschiedenen Halogenatomen, sowie

$R^2$ und $R^3$, welche gleich oder verschieden sein können, für Wasserstoff, für geradkettiges oder verzweigtes Alkyl mit bis zu 4 Kohlenstoffatomen oder für gegebenenfalls einfach oder mehrfach, gleich oder verschieden durch Halogen oder Alkyl mit bis zu 4 Kohlenstoffatomen substituiertes Phenyl stehen,

$R^4$ für geradkettiges oder verzweigtes Alkyl oder Alkoxycarbonyl mit jeweils bis zu 4 Kohlenstoffatomen im Alkylteil, für Cyano oder für gegebenenfalls einfach oder mehrfach, gleich oder verschieden durch Halogen oder Alkyl mit bis zu 4 Kohlenstoffatomen substituiertes Phenyl steht,

Y für Sauerstoff, Schwefel oder die Methylengruppierung steht,

X für Halogen, Cyano, Nitro, für geradkettiges oder verzweigtes Alkyl mit bis zu 4 Kohlenstoffatomen, für Alkoxy, Alkylthio oder Dialkylamino mit 1 oder 2 Kohlenstoffatomen in den jeweiligen Alkylteilen oder für Halogenalkyl mit 1 bis 2 Kohlenstoffatomen und bis zu 5 gleichen oder verschiedenen Halogenatomen steht und

n für eine Zahl von 0 bis 3 steht.

Ganz besonders bevorzugt sind diejenigen Verbindungen der Formel (I), bei denen

$R^1$ für Wasserstoff, Methyl, Ethyl, n- und i-Propyl, für Acetyl, für Methoxycarbonyl oder Ethoxycarbonyl, für gegebenenfalls ein- bis dreifach, gleich oder verschieden substituiertes Phenyl, Benzyl oder Phenylethyl steht,

wobei als jeweilige Phenylsubstituenten insbesondere in Betracht kommen: Fluor, Chlor, Brom, Cyano, Nitro, Methyl, Methoxy, Methylthio, Dimethylamino sowie Trifluormethyl,

$R^2$ und $R^3$, welche gleich oder verschieden sein können, für Wasserstoff, für Methyl oder für gegebenenfalls ein- bis dreifach, gleich oder verschieden durch Chlor oder Methyl substituiertes Phenyl stehen,

$R^4$ für Methyl, Ethyl, n- und i-Propyl, t-Butyl, Cyano, Methoxycarbonyl, Ethoxycarbonyl sowie gegebenenfalls ein- bis dreifach, gleich oder verschieden durch Chlor oder Methyl substituiertes Phenyl steht,

Y für Sauerstoff, Schwefel oder die Methylengruppierung steht,

X für Fluor, Chlor, Brom, Cyano, Nitro, Methyl, Methoxy, Methylthio, Dimethylamino sowie Trifluormethyl steht und

n für eine Zahl von 0 bis 2 steht.

Im einzelnen seien außer den bei den Herstellungsbeispielen genannten Verbindungen die folgenden Verbindungen der allgemeinen Formel (I) genannt:

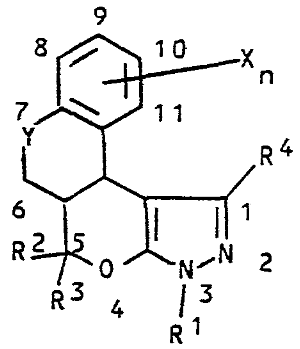

(I)

| $R^1$ | $R^2$ | $R^3$ | $R^4$ | X | Y | n |
|---|---|---|---|---|---|---|
| H | $CH_3$ | $CH_3$ | CN | - | O | 0 |
| H | $CH_3$ | $CH_3$ | $CH_3$ | - | $-CH_2-$ | 0 |
| H | $CH_3$ | $CH_3$ | $CH_3$ | 8,11-$Cl_2$ | S | 2 |
| $CH_3OCO$ | $CH_3$ | $CH_3$ | $CH_3$ | - | O | 0 |
| $C_2H_5OCO$ | $CH_3$ | $CH_3$ | $CH_3$ | - | O | 0 |
| $C_2H_5OCO$ | $CH_3$ | $CH_3$ | $CH_3$ | - | S | 0 |
| H | $CH_3$ | $CH_3$ | $CH_3$ | 10-$NO_2$ | $-CH_2-$ | 1 |
| H | $CH_3$ | $CH_3$ | $CH_3$ | 8,11-$Cl_2$ | $-CH_2-$ | 2 |

Verwendet man beispielsweise 2-(3-Methyl-2-butenyloxy)-benzaldehyd und 3-Methyl-pyrazolin-5-on als Ausgangsstoffe, so kann der Reaktionsablauf des erfindungsgemäßen Verfahrens durch das folgende Formelschema wiedergegeben werden:

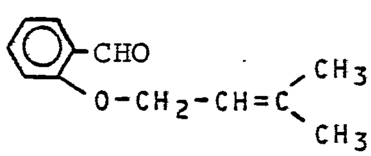

+

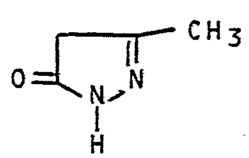

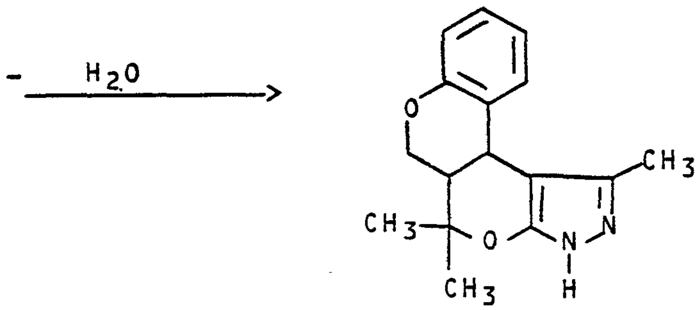


Die zur Durchführung des erfindungsgemäßen Verfahrens benötigten substituierten aromatischen Aldehyde sind durch die Formel (II) allgemein definiert.

Die substituierten aromatischen Aldehyde der Formel (II) sind bekannt /vgl. z.B.: Chem. Pharm. Bull. 27, 2943 (1979) oder Belg. Pat. Nr. 816463 oder Liebigs Ann. Chem. 401, 21 (1913)7.

Die weiterhin zur Durchführung des erfindungsgemäßen Verfahrens benötigten Pyrazolinone sind durch die Formel (III) allgemein definiert.

Die Pyrazolinone der Formel (III) sind ebenfalls bekannt (vgl. z.B. "The Chemistry of Heterocyclic Compounds" Vol. 20, Wiley, New York 1964).

Als Verdünnungsmittel kommen für das erfindungsgemäße Verfahren inerte organische Lösungsmittel infrage. Hierzu gehören vorzugsweise aromatische Kohlenwasserstoffe, wie beispielsweise Benzol, Toluol, Xylol oder Tetralin.

Die erfindungsgemäße Umsetzung kann in Gegenwart eines Katalysators vorgenommen werden. Man kann alle üblicherweise verwendbaren sauren und insbesondere basischen

Katalysatoren einsetzen. Hierzu gehören besonders bevorzugt organische Stickstoffbasen, wie beispielsweise Piperidin, Pyridin, Morpholin oder Triethylamin.

Die Reaktionstemperaturen können bei dem erfindungsgemäßen Verfahren in einem größeren Bereich variiert werden. Im allgemeinen arbeitet man bei Temperaturen zwischen 80°C und 200°C, vorzugsweise bei der Siedetemperatur des zu verwendenden Lösungsmittels.

Bei der Durchführung des erfindungsgemäßen Verfahrens setzt man auf 1 Mol Pyrazolinon der Formel (III) im allgemeinen 1,0 bis 1,3 Mol, vorzugsweise 1,1 bis 1,2 Mol, des substituierten aromatischen Aldehyds der Formel (II) ein.

Man erhitzt die Ausgangsverbindungen zusammen mit 1 bis 3 g des Katalysators in dem jeweiligen Verdünnungsmittel über einem Wasserabscheider, bis die abgeschiedene Wassermenge konstant bleibt.

Zur Isolierung der Verbindungen der Formel (I) filtriert man das erkaltete Reaktionsgemisch und kristallisiert den so erhaltenen Feststoff gegebenenfalls aus einem geeigneten Lösungsmittel um.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So werden z.B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Botrytis-Arten, wie z.B. gegen den Erreger des Grauschimmels (Botrytis cinerea) eingesetzt werden. Daneben können sie auch zur Bekämpfung von Reiskrankheiten, wie z.B. Pellicularia sasakii, eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffe und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut,

vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

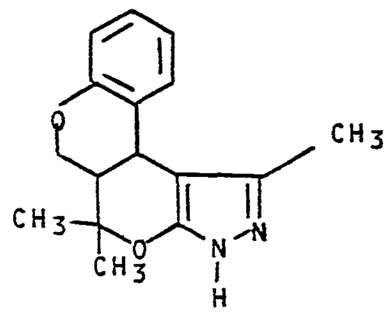

29,4 g (0,3 Mol) 3-Methylpyrazolin-5-on und 62,7 g (0,33 Mol) 2-(3-Methyl-2-butenyloxy)-benzaldehyd werden zusammen mit 1 ml Piperidin in 600 ml absolutem Toluol 15 Stunden über einem Wasserabscheider am Rückfluß gekocht. Nach beendeter Reaktion wird die Mischung im Eisbad abgekühlt und die ausgefallenen Kristalle abgesaugt. Das Rohprodukt wird aus Ethanol umkristallisiert. Nach dem Trocknen erhält man 51,4 g (63 % der Theorie) an 1,5,5-Trimethyl-5a,11b-dihydro-3H,5H,6H,4,7-dioxa-2,3-diazacyclopenta-/c̲7-phenanthren vom Schmelzpunkt 271°C.

Herstellung des Ausgangsproduktes

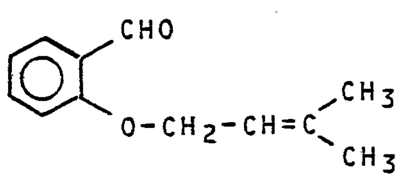

Zu einer Suspension von 60 g (2 Mol) 80 %-igem Natriumhydrid in 2000 ml absolutem Dimethylformamid werden unter Kühlung und Rühren 244 g (2 Mol) Salicylaldehyd in 400 ml Dimethylformamid getropft. Nach einer Stunde bei Raumtemperatur werden wiederum bei 0°C 312,9 g (2,1 Mol) Dimethylallylbromid tropfenweise zugefügt. Die Mischung wird über Nacht bei Raumtemperatur gerührt, danach das Lösungsmittel weitgehend abgedampft und der Rückstand vorsichtig hydrolysiert. Man extrahiert dreimal mit je 400 ml Dichlormethan und wäscht die vereinigten organischen Phasen zweimal mit je 400 ml Wasser. Nach dem Trocknen über Natriumsulfat und Abziehen des Lösungsmittels wird der verbleibende Rückstand im Vakuum destilliert. Man erhält 334,4 g (88 % der Theorie) an 2-(3-Methyl-2-butenyloxy)-benzaldehyd vom Siedepunkt 110°C/ 1.0 mm Hg.

In entsprechender Weise erhält man die folgenden Verbindungen der allgemeinen Formel (I):

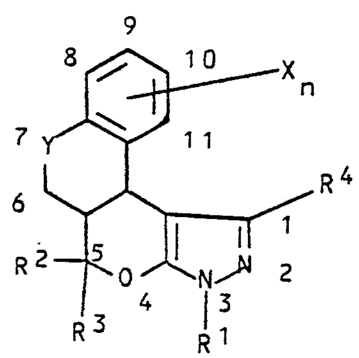

(I)

| Beispiel Nr. | $R^1$ | $R^2$ | $R^3$ | $R^4$ | X | Y | n | physikal. Eigenschaften /Schmelzpunkt °C/ |
|---|---|---|---|---|---|---|---|---|
| 2 | 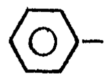 | $CH_3$ | $CH_3$ | $CH_3$ | - | 0 | 0 | 152 |
| 3 | 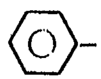 | H | H | $CH_3$ | - | 0 | 0 | 149-50 |
| 4 | $CH_3$ | $CH_3$ | $CH_3$ | $CH_3$ | - | 0 | 0 | 143-45 |
| 5 | 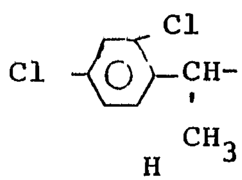 | $CH_3$ | $CH_3$ | $CH_3$ | - | 0 | 0 | 168-70 |
| 6 | H | $CH_3$ | $CH_3$ | $CH_3$ | 9-$OCH_3$ | 0 | 1 | >280 |
| 7 | H | $CH_3$ | $CH_3$ | $CH_3$ | 8,10-$Cl_2$ | 0 | 2 | >280 |
| 8 | H | $CH_3$ | $CH_3$ | $CH_3$ | 9,10-$(OCH_3)_2$ | 0 | 2 | 239-42 |
| 9 | H | $CH_3$ | $CH_3$ | $CH_3$ | 8-$OCH_3$ | 0 | 1 | 237 |
| 10 | H | $CH_3$ | $CH_3$ | $CH_3$ | 10-$NO_2$ | 0 | 1 | >260 |

| Beispiel Nr. | $R^1$ | $R^2$ | $R^3$ | $R^4$ | X | Y | n | physikal. Eigenschaften /Schmelzpunkt °C7 |
|---|---|---|---|---|---|---|---|---|
| 11 | $CH_3$-CO- | $CH_3$ | $CH_3$ | $CH_3$ | - | 0 | 0 | 153-54 |
| 12 | $CH_3$-CO- | H | H | $CH_3$ | - | 0 | 0 | 164-66 |
| 13 | $CH_3$-CO- | $CH_3$ | $CH_3$ | $CH_3$ | 8,11-$Cl_2$ | 0 | 0 | 203 |
| 14 | H | $C_6H_5$ | H | $CH_3$ | - | 0 | 0 | >280 |
| 15 | $CH_3$-CO- | $C_6H_5$ | H | $CH_3$ | - | 0 | 0 | 183-85 |
| 16 | H | $CH_3$ | H | $CH_3$ | - | 0 | 0 | 199-200 |
| 17 | H | $CH_3$ | $CH_3$ | $COOC_2H_5$ | - | 0 | 0 | 128-32 |
| 18 | H | $CH_3$ | $CH_3$ | $CH_3$ | - | S | 0 | 256 |
| 19 | H | $CH_3$ | $CH_3$ | $(CH_3)_3C$- | - | 0 | 0 | 238-40 |

| Beispiel Nr. | $R^1$ | $R^2$ | $R^3$ | $R^4$ | X | Y | n | physikal. Eigenschaften /Schmelzpunkt °C7 |
|---|---|---|---|---|---|---|---|---|
| 20 | H | $CH_3$ | $CH_3$ | $C_6H_5$ | - | 0 | 0 | 235-37 |
| 21 | H | $CH_3$ | $CH_3$ | $(CH_3)_2CH-$ | - | 0 | 0 | 258-60 |
| 22 | $CH_3-CO$ | $CH_3$ | $CH_3$ | $CH_3$ | - | S | 0 | 142-143 |
| 23 | H | $CH_3$ | $CH_3$ | $CH_3$ | 10-Br | 0 | 0 | > 260 |
| 24 | $C_2H_5O-CO-$ | $CH_3$ | $CH_3$ | $CH_3$ | - | 0 | 0 | 104 |
| 25 | $CH_3O-CO-$ | $CH_3$ | $CH_3$ | $CH_3$ | - | 0 | 0 | 171 |
| 26 | H | $CH_3$ | $CH_3$ | $CH_3$ | $9-N(C_2H_5)_2$ | 0 | 1 | 230 (Zers.) |

Anwendungsbeispiele

In dem folgenden Anwendungsbeispiel wird die nachstehend aufgeführte Verbindung als Vergleichssubstanz eingesetzt:

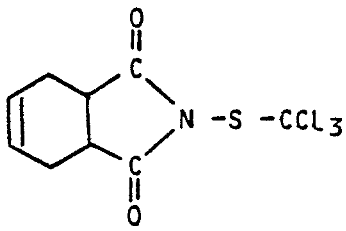

(A)

N-Trichlormethylthio-tetrahydrophthalimid

Beispiel A

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß folgender Herstellungsbeispiele: 1, 6, 7, 9, 10 und 11.

Patentansprüche

1. Pyrano-pyrazol-Derivate der allgemeinen Formel (I)

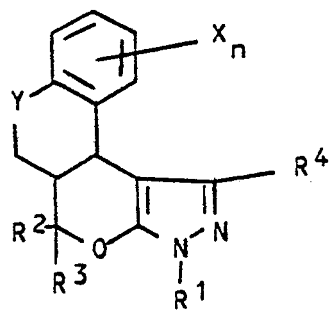

(I)

in welcher

$R^1$ für Wasserstoff, für Alkyl, für Alkanoyl, für Alkoxycarbonyl, für gegebenenfalls substituiertes Phenyl oder für gegebenenfalls substituiertes Phenylalkyl steht,

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls substituiertes Phenyl steht,

Y für Sauerstoff, Schwefel oder die Methylengruppe steht,

X für Halogen, Alkyl, Alkoxy, Alkylthio,

Halogenalkyl, Dialkylamino, Nitro oder Cyano steht und

n für eine Zahl von 0 bis 3 steht.

2. Pyrano-pyrazol-Derivate gemäß Anspruch 1, wobei in der Formel (I)

$R^1$ für Wasserstoff, für geradkettiges oder verzweigtes Alkyl, Alkanoyl oder Alkoxycarbonyl mit jeweils bis zu 4 Kohlenstoffatomen im Alkylteil, für gegebenenfalls einfach oder mehrfach, gleich oder verschieden substituiertes Phenyl oder für gegebenenfalls einfach oder mehrfach, gleich oder verschieden substituiertes Phenylalkyl mit 1 oder 2 Kohlenstoffatomen im Alkylteil steht, wobei als Phenylsubstituenten jeweils in Betracht kommen: Halogen, Cyano, Nitro, Alkyl mit bis zu 4 Kohlenstoffatomen, Alkoxy, Alkylthio oder Dialkylamino mit 1 oder 2 Kohlenstoffatomen in den jeweiligen Alkylteilen, sowie Halogenalkyl mit 1 oder 2 Kohlenstoffatomen und bis zu 5 gleichen oder verschiedenen Halogenatomen, sowie

$R^2$ und $R^3$, welche gleich oder verschieden sein können, für Wasserstoff, geradkettiges oder verzweigtes Alkyl mit bis zu 4

Kohlenstoffatomen oder für gegebenenfalls einfach oder mehrfach, gleich oder verschieden durch Halogen oder Alkyl mit bis zu 4 Kohlenstoffatomen substituiertes Phenyl stehen,

$R^4$ für geradkettiges oder verzweigtes Alkyl oder Alkoxycarbonyl mit jeweils bis zu 4 Kohlenstoffatomen im Alkylteil, für Cyano oder für gegebenenfalls einfach oder mehrfach, gleich oder verschieden durch Halogen oder Alkyl mit bis zu 4 Kohlenstoffatomen substituiertes Phenyl steht,

Y für Sauerstoff, Schwefel oder die Methylengruppierung steht,

X für Halogen, Cyano, Nitro, für geradkettiges oder verzweigtes Alkyl mit bis zu 4 Kohlenstoffatomen, für Alkoxy, Alkylthio oder Dialkylamino mit 1 bis 2 Kohlenstoffatomen in den jeweiligen Alkylteilen oder für Halogenalkyl mit 1 oder 2 Kohlenstoffatomen und bis zu 5 gleichen oder verschiedenen Halogenatomen steht und

n für eine Zahl von 0 bis 3 steht.

3. Pyrano-pyrazol-Derivate gemäß Anspruch 1, wobei in der Formel (I)

$R^1$ für Wasserstoff, Methyl, Ethyl, n- und i-Propyl, für Acetyl, für Methoxycarbonyl oder Ethoxycarbonyl, für gegebenenfalls ein- bis dreifach, gleich oder verschieden substituiertes Phenyl, Benzyl oder Phenylethyl steht, wobei als jeweilige Phenylsubstituenten in Betracht kommen: Fluor, Chlor, Brom, Cyano, Nitro, Methyl, Methoxy, Methylthio, Dimethylamino sowie Trifluormethyl,

$R^2$ und $R^3$, welche gleich oder verschieden sein können, für Wasserstoff, für Methyl oder für gegebenenfalls ein- bis dreifach, gleich oder verschieden durch Chlor oder Methyl substituiertes Phenyl stehen,

$R^4$ für Methyl, Ethyl, n- und i-Propyl, t-Butyl, Cyano, Methoxycarbonyl, Ethoxycarbonyl sowie gegebenenfalls ein- bis dreifach, gleich oder verschieden durch Chlor oder Methyl substituiertes Phenyl steht,

Y für Sauerstoff, Schwefel oder die Methylengruppierung steht,

X für Fluor, Chlor, Brom, Cyano, Nitro, Methyl, Methoxy, Methylthio, Dimethylamino sowie Trifluormethyl steht und

n für eine Zahl von 0 bis 2 steht.

4. Verfahren zur Herstellung von Pyrano-pyrazol-Derivaten der Formel (I)

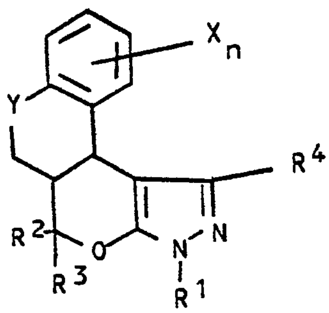

(I)

in welcher

$R^1$ für Wasserstoff, für Alkyl, für Alkanoyl, für Alkoxycarbonyl, für gegebenenfalls substituiertes Phenyl oder für gegebenenfalls substituiertes Phenylalkyl steht,

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls substituiertes Phenyl steht,

Y für Sauerstoff, Schwefel oder die Methylengruppe steht,

X für Halogen, Alkyl, Alkoxy, Alkylthio, Halogenalkyl, Dialkylamino, Nitro oder Cyano steht und

n für eine Zahl von 0 bis 3 steht,

dadurch gekennzeichnet, daß man substituierte aromatische Aldehyde der allgemeinen Formel (II),

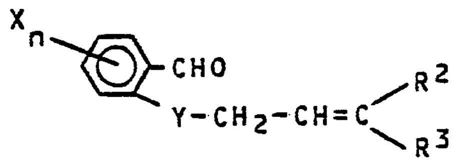

(II)

in welcher

$R^2$, $R^3$, X, Y und n die oben angegebene Bedeutung haben,

mit Pyrazolinonen der allgemeinen Formel (III),

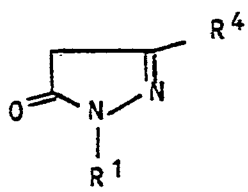

(III)

in welcher

$R^1$ und $R^4$ die oben angegebenene Bedeutung haben,

in Gegenwart eines Verdünnungsmittels und gegebenenfalls in Gegenwart eines Katalysators umsetzt.

5. Pflanzenschutzmittel, gekennzeichnet durch einen Gehalt an mindestens einem substituierten Pyrano-pyrazol-Derivat der Formel (I) in Ansprüchen 1 und 4.

6. Verfahren zur Bekämpfung von Pilzen, dadurch gekennzeichnet, daß man substituierte Pyrano-pyrazol-Derivate der Formel (I) in Ansprüchen 1 und 4 auf Pilze oder ihren Lebensraum einwirken läßt.

7. Verwendung von substituierten Pyrano-pyrazol-Derivaten der Formel (I) in Ansprüchen 1 und 4 als Pflanzenschutzmittel.

8. Verwendung von substituierten Pyrano-pyrazol-Derivaten der Formel (I) in Ansprüchen 1 und 4 zur Bekämpfung von Pilzen.

9. Verfahren zur Herstellung von Pflanzenschutzmitteln, dadurch gekennzeichnet, daß man substituierte Pyrano-pyrazol-Derivate der Formel (I) in Ansprüchen 1 und 4 mit Streckmitteln und/oder oberflächenaktiven Mitteln vermischt.

Europäisches Patentamt

# EUROPÄISCHER RECHERCHENBERICHT

0110243

Nummer der Anmeldung

EP 83 11 1477

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 001 428 (BAYER AG)<br>* Insgesamt *<br>--- | 1,5,7,9 | C 07 D 491/153<br>C 07 D 495/14<br>A 01 N 43/90 //<br>C 07 C 47/575<br>(C 07 D 491/153<br>C 07 D 311/00<br>C 07 D 311/00<br>C 07 D 231/00 )<br>(C 07 D 495/14<br>C 07 D 335/00<br>C 07 D 311/00<br>C 07 D 231/00 ) |
| A | ANGEWANDTE CHEMIE INTERNATIONAL EDITION ENGL., Band 21, Nr. 11, November 1982, Seiten 863-864, Verlag Chemie GmbH., Weinheim, DE<br>LUTZ-F. TIETZE u.a.: "Control of the conformation of transition states in intramolecular Diels-Alder reactions with inverse electron demand" * Insgesamt *<br>----- | 1,4 | RECHERCHIERTE SACHGEBIETE (Int. Cl. 3)<br>C 07 D 491/00<br>C 07 D 495/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 16-02-1984 | ALLARD M.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument